# EUROPÄISCHE PATENTSCHRIFT

(11) **EP 0 268 975 B1**
(45) Veröffentlichungstag und Bekanntmachung des Hinweises auf die Patenterteilung: **25.05.1994**
(21) Anmeldenummer: 87116920.7
(22) Anmeldetag: 17.11.1987
(51) Int. Cl.: C07D 265/02, C07D 209/42, C07D 209/52, C07D 209/96, C07D 207/16, A61K 31/40

(54) **Verfahren zur Herstellung von mono-, bi- und tricyclischen Aminosäuren, Zwischenprodukte dieses Verfahrens sowie ein Verfahren zu deren Herstellung**
Process for the preparation of mono, bi and tricyclic amino acids, intermediary compounds and their preparation
Procédé de préparation d'acides aminés mono-, bi- et tricycliques, produits intermédiaires et leur préparation

(30) Priorität: 21.11.1986 DE 3639879
(43) Veröffentlichungstag der Anmeldung: 01.06.1988
(73) Patentinhaber: HOECHST AKTIENGESELLSCHAFT, 65926 Frankfurt am Main (DE)
(72) Erfinder: Henning, Rainer, Dr., D-6234 Hattersheim am Main (DE); Urbach, Hansjörg, Dr., D-6242 Kronberg/Taunus (DE)

(56) Entgegenhaltungen:
- EP-A- 0 132 580
- JOURNAL OF THE CHEMICAL SOCIETY CHEMICAL COMMUNICATIONS, 1979 T.L. GILCHRIST et al. "Ethyl 3-Bromo-2-hydroxyiminopro- panoate, a Reagent for the Preparation of Ethyl Esters of alpha-Amino Acids" Seiten 1089-1090
- JOURNAL OF THE CHEMICAL SOCIETY CHEMICAL COMMUNICATIONS, 1979 T.L. GILCHRIST et al. "Decarboxylation and Fragmentation of 5,6-Dihydro- -4H-1,2-oxazine-3-carboxylic Acids" Seiten 1090-1091
- SYNTHESIS 1989 (Seiten 265-268)

## Beschreibung

Prolinderivate der Formel I sind aus der Literatur bekannt. Ein Verfahren zu ihrer Herstellung ist in EP-A 132 580 beschrieben.
Überraschenderweise wurde nun ein Verfahren zur Herstellung von Prolinderivaten gefunden, daß sich besonders durch seine einfache Durchführbarkeit auszeichnet.

Die Erfindung betrifft ein Verfahren zur Herstellung von Verbindungen der Formel I 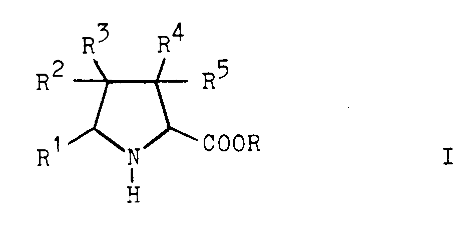
in welcher
- R: für Wasserstoff, (C₁-C₆)-Alkyl oder (C₇-C₉)-Aralkyl steht, und
- R¹ bis R⁵: gleich oder verschieden sind und unabhängig voneinander Wasserstoff, (C₁-C₈)-Alkyl, (C₃-C₉)-Cycloalkyl, (C₃-C₉)-Cycloalkyl-(C₁-C₄)-alkyl, (C₅-C₉)-Cycloalkenyl-(C₁-C₄)-alkyl, (C₆-C₁₂)-Aryl-(C₁-C₄)-alkyl oder (C₆-C₁₂)-Aryl, wobei die beiden letztgenannten Substituenten jeweils im Arylteil durch (C₁-C₄)-Alkyl, (C₁-C₄)-Alkoxy, Hydroxy, Halogen, Nitro, Methylendioxy und/oder Cyano mono-, di- oder trisubstituiert sein können, bedeuten
oder in welcher
jeweils die Reste R¹ und R², R² und R³ sowie R⁴ und R⁵ zusammen mit dem sie tragenden Kohlenstoffatom bzw. mit den beiden sie tragenden Kohlenstoffatomen ein 4- bis 10-gliedriges gesättigtes oder ungesättigtes mono- oder bicyclisches carbocyclisches Ringsystem bilden und die übrigen Reste Wasserstoff sind,
das dadurch gekennzeichnet ist, daß man eine Verbindung der Formel II 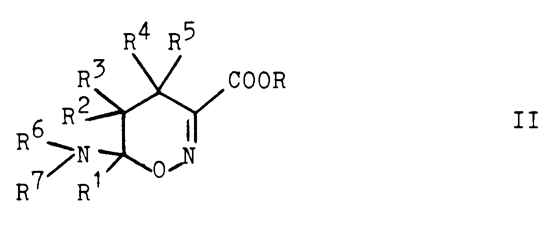
in welcher
- R und R¹ bis R⁵: gleich oder verschieden sind und die obengenannte Bedeutung haben, und
- R⁶ und R⁷: (C₁-C₆)-Alkyl, (C₇-C₉)-Aralkyl bedeuten, oder
- R⁶ und R⁷: zusammen mit dem sie tragenden Stickstoffatom einen 5- bis 10-gliedrigen Heterocyclus bilden, der zusätzlich ein Sauerstoffatom enthalten kann,
mit einem Reduktionsmittel behandelt.

Als Reduktionsmittel kommen in Betracht:
Wasserstoff in Gegenwart eines Katalysators wie Palladiummohr, Palladium auf Aktivkohle, Platin auf Aktivkohle, Rhodium auf Aktivkohle, Raney-nickel oder Raney-Kobalt in einem niederen Alkohol (wie (C₁-C₄)-Alkanol) oder einer niederen Carbonsäure (wie Ameisensäure, Essigsäure, Propionsäure oder Buttersäure) mit oder ohne Zusatz einer Mineralsäure wie z.B. Chlorwasserstoffsäure, Bromwasserstoffsäure oder Schwefelsäure sowie Natriumborhydrid, Natriumcyanborhydrid, sulfitiertes Natriumborhydrid (NaBH₂S₃), Boran-Dimethylsulfid, Boran-Pyridin, Boran-Trimethylamin, Natriumdithionit, Natrium in einem niederen Alkohol (wie (C₁-C₄)-Alkanol), Natriumamalgam oder Aluminiumamalgam, vorzugsweise jedoch Reduktionsmittel wie Wasserstoff in Gegenwart eines Katalysators wie Palladiummohr, Palladium auf Aktivkohle, Platin auf Aktivkohle, Rhodium auf Aktivkohle, Raney-nickel oder Raney-Kobalt.

Die Synthese kann zwischen -20°C und dem Siedepunkt des Reaktionsgemisches, vorzugsweise zwischen 20°C und 60°C durchgeführt werden. Der H₂-Druck beträgt bei der Reaktion 1·10⁵ bis 2·10⁷ N·m⁻², vorzugsweise 1·10⁵ bis 5·10⁶ N·m⁻².

Eine bevorzugte Ausführungsform ist dadurch gekennzeichnet, daß Verbindungen der Formel I hergestellt werden, in welcher
- R: die obengenannte Bedeutung hat,
- R¹ bis R⁵: gleich oder verschieden sind und unabhängig voneinander Wasserstoff, Methyl, Ethyl, Propyl, Isopropyl, tert. Butyl, n-Pentyl, Cyclopentyl, Cyclohexyl, Cycloheptyl, Cyclopentylmethyl, Cyclopentylethyl, Cyclohexylmethyl, Phenyl, Napthyl, 4-Methoxyphenyl, 4-Fluorphhenyl, 4-Chlorphenyl, 3,4-Dimethoxyphenyl, 3,4-Methylendioxyphenyl, 3,4-Dichlorphenyl, p-Tolyl, Benzyl, 4-Methoxybenzyl, 4-Chlorbenzyl, Phenethyl, 2-Phenylpropyl oder 1-Phenylpropyl bedeuten,
oder in welcher
jeweils die Reste R¹ und R², R² und R³ sowie R⁴ und R⁵ in der oben definierten Weise einen Cyclobutan-, Cyclopentan-, Cyclohexan-, Cycloheptan-, Cyclooctan-, Bicyclo[2.2.1]heptan- oder Bicyclo[2.2.2]octan-Ring bilden, wobei die übrigen Reste Wasserstoff sind.

Besonders bevorzugt ist eine Ausführungsform, die dadurch gekennzeichnet ist, daß Verbindungen der Formel I hergestellt werden, in welcher
- R: die obengenannte Bedeutung hat, insbesondere aber Wasserstoff, tert. Butyl oder Benzyl bedeutet und worin
einer oder zwei der Reste R¹ bis R⁵ unabhängig voneinander Methyl, Ethyl, Propyl, Isopropyl, n-Pentyl, Cyclopentyl, Cyclohexyl, Phenyl, 4-Methoxyphenyl, 4-Fluorphenyl, Benzyl, Phenethyl oder 4-Methoxybenzyl und die übrigen Wasserstoff bedeuten, oder
jeweils die Reste R¹ und R² sowie R² und R³ mit dem sie tragenden Kohlenstoffatom bzw. mit den beiden sie tragenden Kohlenstoffatomen einen Cyclopentan-, Cyclohexan-, Cycloheptan-, Bicyclo[2.2.1]heptan- oder Bicyclo[2.2.2]-octan-Ring bilden, wobei die übrigen Reste Wasserstoff bedeuten.

Das erfindungsgemäße Verfahren liefert je nach Durchführung und Art der Substituenten R¹ bis R⁵ die Verbindungen der Formel I als Gemische von Enantiomeren oder Diastereomeren oder als reine Diastereomere. Entstehende Gemische können durch geeignete, an sich bekannte Verfahren wie fraktionierte Kristallisation oder Chromatographie für Diastereomere, oder Bildung von diastereomeren Salzen, gegebenenfalls von geeigneten Derivaten, für Enantiomerengemische in die Bestandteile getrennt werden.

Ganz besonders vorteilhaft können nach dem erfindungsgemäßen Verfahren die folgenden Verbindungen der Formel I hergestellt werden.
cis-Octahydro[1H]indol-2-exo-carbonsäure-ethylester
cis-Octahydro[1H]indol-2-endo-carbonsäure-ethylester
trans-Octahydro[1H]indol-2-α-carbonsäure-ethylester
trans-Octahydro[1H]indol-2-β-carbonsäure-ethylester
cis-Octahydrocyclopenta[b]pyrrol-2-exo-carbonsäure-ethylester
cis-Octahydrocyclopenta[b]pyrrol-2-endo-carbonsäure-ethylester
trans-Octahydrocyclopenta[b]pyrrol-2-α-carbonsäure-ethylester
trans-Octahydrocyclopenta[b]pyrrol-2-β-carbonsäure-ethylester
2-Aza-spiro[4,5]decan-3-carbonsäure-ethylester
2-Aza-spiro[4,4]nonan-3-carbonsäure-ethylester
Spiro[bicyclo[2.2.2]octan-2,3ʹ-pyrrolidin]-5-exo-carbonsäure-ethylester
Spiro[bicyclo[2.2.2]octan-2,3ʹ-pyrrolidin]-5-endo-carbonsäure-ethylester
Spiro[bicyclo[2.2.1]heptan-2,3ʹ-pyrrolidin]-5-exo-carbonsäure-ethylester
cis-exo-3-Azatricyclo[5.2.1.0^{2,6}]decan-4-exo-carbonsäure-ethylester
cis-exo-3-Azatricyclo[5.2.1.0^{2,6}]decan-4-endo-carbonsäure-ethylester
cis-endo-3-Azatricyclo[5.2.1.0^{2,6}]decan-4-endo-carbonsäure-ethylester
cis-endo-3-Azatricyclo[5.2.1.0^{2,6}]decan-4-exo-carbonsäure-ethylester
cis-Decahydrocyclohepta[b]pyrrol-2-exo-carbonsäure-ethylester
cis-Decahydrocyclohepta[b]pyrrol-2-endo-carbonsäure-ethylester
trans-Decahydrocyclohepta[b]pyrrol-2-α-carbonsäure-ethylester
trans-Decahydrocyclohepta[b]pyrrol-2-β-carbonsäure-ethylester
1-Aza-spiro[4,5]decan-2-carbonsäure-ethylester
1-Aza-spiro[4,4]nonan-2-carbonsäure-ethylester
4,5-cis-Diethylprolin-ethylester
4,5-cis-Dimethylprolin-ethylester
5,5-Dimethylprolin-ethylester
4,4-Dimethylprolin-ethylester
4,4-Diethylprolin-ethylester
3,3-Dimethylprolin-ethylester
4,5-cis-Diphenylprolin-ethylester
4-Phenylprolin-ethylester
sowie die entsprechenden freien Säuren.

Verbindungen der Formel I, worin R (C₁-C₆)-Alkyl oder (C₇-C₉)-Aralkyl bedeutet, können in an sich bekannter Weise unter hydrogenolytischen, sauren oder basischen Bedingungen, beispielsweise mit Mineralsäure wie Chlorwasserstoffsäure, Bromwasserstoffsäure oder Schwefelsäure bei 0° bis 150°C, vorzugsweise bei 60° bis 120°C oder mit Wasserstoff in Gegenwart eines Übergangsmetallkatalysators, z.B. Raney-Nickel oder Palladium auf Aktivkohle, in Verbindungen der Formel I, worin R wasserstoff bedeutet, überführt werden.

In J. Chem. Soc. Chem. Commun 1979, 1089 ist die Herstellung einer Verbindung der Formel II beschrieben, worin R Ethyl bedeutet, R¹ und R² gemeinsam eine -[CH₂]₄-Kette bilden, R³ bis R⁵ für Wasserstoff und R⁶ und R⁷ mit dem sie tragenden Stickstoffatom für einen Morpholinring stehen. Weitere Umsetzungen sind nicht offenbart.

Mit Ausnahme dieser genannten Verbindung sind die Verbindungen der Formel II neu und ebenfalls Gegenstand der Erfindung.

Die Erfinding betrifft weiterhin ein Verfahren zur Herstellung von Verbindungen der Formel II, dadurch gekennzeichnet, daß man eine Verbindung der Formel III 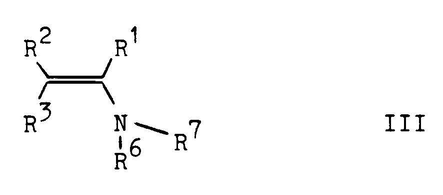
mit einer Verbindung der Formel IV 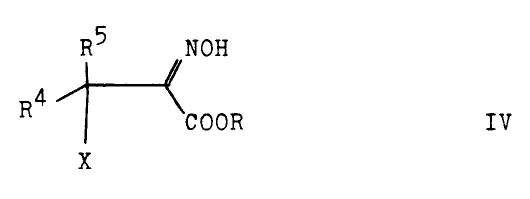
worin
- R: für Wasserstoff, (C₁-C₆)-Alkyl oder (C₇-C₉)-Aralkyl steht,
- R¹ bis R⁵: gleich oder verschieden sind und unabhängig voneinander Wasserstoff, (C₁-C₈)-Alkyl, (C₃-C₉)-Cycloalkyl, (C₃-C₉)-Cycloalkyl-(C₁-C₄)-alkyl, (C₅-C₉)-Cycloakenyl-(C₁-C₄)-alkyl, (C₆-C₁₂)-Aryl-(C₁-C₄)-alkyl oder (C₆-C₁₂)-Aryl, wobei die beiden letztgenannten Substituenten jeweils im Arylteil durch (C₁-C₄)-Alkyl, (C₁-C₄)-Alkoxy, Hydroxy, Halogen, Nitro, Methylendioxy und/oder Cyano mono-, di- oder trisubstituert sein können, bedeuten
oder worin
jeweils die Reste R¹ und R², R² und R³ sowie R⁴ und R⁵ zusammen mit dem sie tragenden Kohlenstoffatom bzw. mit den beiden sie tragenden Kohlenstoffatomen ein 4- bis 10-gliedriges gesättigtes oder ungesättigtes mono- oder bicyclisches carbocyclisches Ringsystem bilden und die übrigen Reste Wasserstoff sind,
- R⁶ und R⁷: (C₁-C₄)-Alkyl, (C₇-C₉)-Aralkyl bedeuten, oder
- R⁶ und R⁷: zusammen mit dem sie tragenden Stickstoffatom einen 5- bis 10-gliedrigen Heterocyclus bilden, der zusätzlich ein Sauerstoffatom enthalten kann, und
- X: Chlor oder Brom bedeutet, umsetzt.

Die Umsetzung wird in einem aprotischen organischen Lösungsmittel, vorzugsweise einem Ether wie z.B. Diethylether, Diisopropylether, Tetrahydrofuran oder Dioxan oder einem chlorierten Kohlenwasserstoff wie z.B. Dichlormethan oder Chloroform in Gegenwart einer schwachen Base wie beispielsweise Natriumhydrogencarbonat, Natriumcarbonat, Kaliumcarbonat oder einem Trialkylamin oder Pyridin in einem Temperaturbereich von -40°C bis 100°C, vorzugsweise von 0° bis 50°C durchgeführt.

Die Verbindungen der Formel III sind aus G. Cook, Enamines (Marcel Decker, New York und London, 1969) bekannt. Verbindungen der Formel IV erhält man aus Verbindungen der Formel V 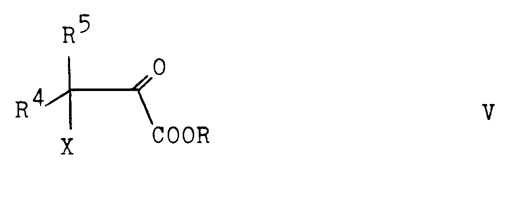
worin
R, R⁴, R⁵ und X wie oben definiert sind, durch Umsetzung mit Hydroxylamin, wie in J. Chem. Soc. Commun 1979, 1089 beschrieben.

Die Verbindungen der Formel I sind wertvolle Zwischenprodukte bei der Herstellung von Pharmazeutika, insbesondere von Inhibitoren der "Angiotensin Converting Enzym" (ACE). Verbindungen dieses Typs sind beispielsweise aus der EP-A-50 800 bekannt oder sind auch Gegenstand der deutschen Patentanmeldung P 31 51 690.4. Solche ACE-Inhibitoren sind beispielsweise substituierte Acylderivate der Formel VI 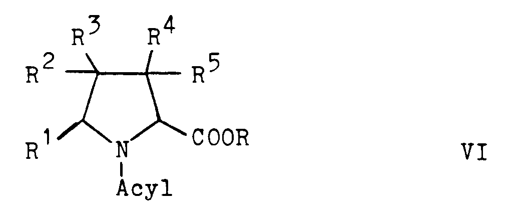
in welcher R, R¹ bis R³ wie vorstehend definiert sind, R⁴ und R⁵ Wasserstoff bedeuten und Acyl z.B. für einen Rest der Formel VII steht, 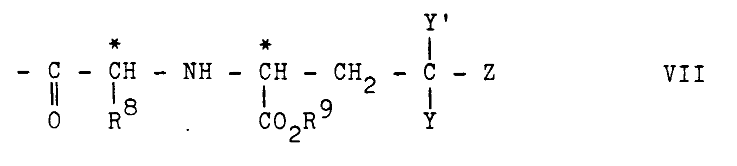
worin
- R⁸: Wasserstoff, (C₁-C₆)-Alkyl, das gegebenenfalls durch Amino, (C₁-C₄)-Acylamino oder Benzoylamino substituiert sein kann, (C₂-C₆)-Alkenyl, (C₅-C₉)-Cycloalkyl, (C₅-C₉)-Cycloalkenyl, (C₅-C₇)-Cycloalkyl-(C₁-C₄)-alkyl, Aryl oder teilhydriertes Aryl, das jeweils durch (C₁-C₂)-Alkyl, (C₁-C₂)-Alkoxy oder Halogen substituiert sein kann, Aryl-(C₁-C₄)-alkyl, dessen Arylrest, wie vorstehen definiert, substituiert sein kann, einen mono- bzw. bicyclischen Heterocyclenrest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen oder eine Seitenkette einer Aminosäure,
- R⁹: Wasserstoff, (C₁-C₆)-Alkyl, (C₂-C₆)-Alkenyl oder Aryl-(C₁-C₄)-alkyl,
- Y und Yʹ: Wasserstoff oder zusammen Sauerstoff,
- Z: (C₁-C₆)-Alkyl, (C₂-C₆)-Alkenyl, (C₅-C₉)-Cycloalkyl, Aryl, das durch (C₁-C₄)-Alkyl, (C₁-C₄)-Alkoxy, Hydroxy, Halogen, Nitro, Amino, (C₁-C₄)-Alkylamino, Di(C₁-C₄)-alkylamino und/oder Methylendioxy mono-, di-oder trisubstituiert sein kann, oder Indol-3-yl bedeuten
sowie deren physiologisch unbedenkliche Salze.

Verbindungen der Formel VI können beispielsweise durch N-Acylierung von geeigneten Estern der Verbindungen der Formel I, wie beispielsweise Benzyl- oder tert.-Butylestern mit Verbindungen der Formel Acyl-OH, in der Acyl wie vorstehend definiert ist und anschließend hydrogenolytische, saure oder basische Abspaltung der Estergruppen hergestellt werden.

Die Kondensation von Estern der Verbindungen der Formel I mit Verbindungen der Formel Acyl-OH erfolgt vorzugsweise nach bekannten Methoden der Peptidchemie. Besonders bevorzugt sind solche Verfahren, die ausreichend Schutz vor Racemisierung bieten, wie z.B. die DCC/HOBt-Methode oder die im US-Patent 43 31 592 beschriebene Alkanphosphonsäureanhydridmethode.

Die Verbindungen der Formel VI besitzen eine lang andauernde, intensive blutdrucksenkende Wirkung. Sie werden nach peroraler Gabe gut resorbiert und können zur Bekämpfung des Bluthochdrucks verschiedener Genese eingesetzt und für sich oder in Kombination mit anderen blutdrucksenkenden, gefäßerweiternden oder diuretisch wirksamen Verbindungen angewandt werden. Die Anwendung kann intravenös, subkutan oder peroral erfolgen, die perorale Gabe wird bevorzugt. Die Dosierung liegt bei peroraler Gabe in der Regel bei 0,01 bis 10 mg/kg Tag.
Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden. Bei intravenöser und subkutaner Gabe sollte Einzeldosis zwischen 0,1 und 250 µ/Tag liegen.

Die folgenden Beispiele sollen die Erfindung erläutern, ohne sie auf die beschriebenen Beispiele zu beschränken.

### Beispiel 1

### cis-Octahydro[1H]indol-2-carbonsäure-ethylester

### a) 8a-Morpholino-1-oxa-2-aza-1,4,4a,5,6,7,8,8a-octahydronaphthalin-3-carbonsäureethylester

5 g (30 mmol) Morpholino-cyclohexen und 1,9 g (10 mmol) 3-Bromo-2-hydroxyiminopropionsäure-ethylester werden zusammen mit 2 g Kaliumcarbonat in 80 ml Dichlormethan 4 Stunden bei Raumtemperatur gerührt. Nach dem Abfiltrieren wird eingeengt und an Kieselgel chromatographiert (Laufmittel: Essigester/Cyclohexan 1:4). Man erhält 2,1 g farbloses Öl.
- ¹H-NMR (CDCl₃):: δ = 4,3 (q,2H); 3,6 (t,4H); 2,8 (m,2H); 2,6 (m,2H); 2,3 (m,2H); 1,4 (t,3H); 1,7 - 1,0 (m,9H) ppm.

### b) cis-Octahydro[1H]indol-2-carbonsäure-ethylester

2,1 g des Produkts aus Beispiel 1a) werden in 60 ml Ethanol mit 0,5 g Raney-Nickel 4 Stunden bei 50°C unter Normaldruck hydriert. Man erhält 1,3 g cis-Octahydro[1H]indol-2-carbonsäure-ethylester als blaßgelbes Öl nach dem Abfiltrieren und Einengen im Vakuum.

### Beispiel 2

### cis-2-Aza-bicyclo[3.3.0]octan-3-carbonsäure-methylester

### a) 1-Morpholino-2-oxa-3-aza-bicyclo[4.3.0]non-3-en-4-carbonsäure-methylester

4,6 g (30 mmol) Morpholino-cyclopentan werden analog der in Beispiel 1a) angegebenen Vorschrift mit 1,75 g (10 mmol) 3-Bromo-hydroxyimino-propionsäure-methylester umgesetzt. Man erhält 1,9 g farbloses Öl.
- ¹H-NMR (CDCl₃):: δ = 3,8 (s,3H); 3,6 (t,4H); 2,8 (m,2H); 2,6 (m,2H); 2,3 (m,2H); 1,7-1,0 (m,3H) ppm.

### b) cis-2-Aza-bicyclo[3.3.0]octan-3-carbonsäure-methylester

Analog der in Beispiel 1b) angegebenen Vorschrift erhält man aus 1,9 g 1-Morpholino-2-oxa-3-aza-bicyclo[4.3.0]non-3-en-4-carbonsäure-methylester 1,0 g cis-2-Aza-bicyclo[3.3.0]octan-3-carbonsäure-methylester als blaßgelbes Öl.

### Beispiel 3

### Spiro[bicyclo[2.2.2]octan-2,3ʹ-pyrrolidin]-5ʹ-carbon-säure-ethylester

### a) Spiro[bicyclo[2.2.2]octan-2,5ʹ-(6ʹ-pyrrolidino)-4ʹH-5ʹ,6ʹ-dihydro-1ʹ-2ʹ-oxazin]

4,5 g (20 mmol) 2-Pyrrolidinomethylen-bicyclo[2.2.2]octan und 2,75 g (13 mmol) 3-Bromo-2-hydroxyimino-propionsäure-ethylester werden in 50 ml Dichlormethen gelöst. Anschlißend werden 5 g Natriumcarbonat zugegeben und 2 Stunden bei 25°C gerührt. Nach der Filtration wird eingeeingt. Die Chromatographie an Kieselgel mit Essigester/Cyclohexan (1:8) als Laufmittel ergibt 2,45 g Produkt. Fp. 116-118°C (n-Hexan)
- ¹H-NMR (CDCl₃):: δ = 4,95 + 4,85 (2d,1H); 4,3 (2q,2H); 3,0 (m,2H); 2,7 (m,2H); 2,6 + 2,2 (AB-System,2H); 1,9 - 1,4 (m,16H); 1,4 (t,3H) ppm.

### b) Spiro[bicyclo[2.2.2]octan-2,3ʹ-pyrrolidin]-5ʹ-carbon-säure-ethylester

2,4 g des Produktes aus Beispiel 3a) werden in 60 ml Ethanol mit 0,4 g Raney-Nickel analog der in Beispiel 1b) angegebenen Vorschrift hydriert. Man erhält 1,9 g eines blaßgelben Öls, das die beiden Diastereomeren (S*,S* bzw. S*,R*) im Verhältnis von 3:1 enthält. Die Isomeren können nach der Acetylierung mit Acetylchlorid/Triethylamin in Tetrahydrofuran getrennt werden.
- Isomer 1 (S,S):: 0,5 g, Fp. 122°C
- ¹H-NMR (CDCl₃):: δ = 4,4 (m,1H); 4,2 (q,2H); 3,9 - 3,1 (m,1H); 2,4 - 1,8 (m,1H); 2,1 + 1,95 (2s,3H); 1,7 - 1,3 (m,13H); 1,25 (t,3H) ppm.
- Isomer 2 (S,R):: 1,5 g, Öl
- ¹H-NMR (CDCl₃):: δ = 4,5 - 4,3 (m,1H); 4,3 - 4,1 (m,2H); 3,6 - 3,3 (m,2H); 2,4 - 1,8 (m,2H); 2,1 + 1,95 (2s,3H); 1,8 - 1,3 (m,12H); 1,3 (t,3H) ppm.

### Beispiel 4

### 4,4-Pentamethylen-prolin-ethylester

### a) 3-Ethoxycarbonyl-5,5-pentamethylen-6-pyrrolidino-4H-5,6-dihydro-1,2-oxazin

3,3 g (20 mmol) Pyrrolidinomethylen-cyclohexan werden analog der in Beispiel 3a) angegebenen Vorschrift mit 2,75 g (13 mmol) 3-Bromo-2-hydroxyimino-propionsäure-ethylester umgesetzt. Man erhält 1,6 g 3-Ethoxycarbonyl-5,5-pentamethylen-6-pyrrolidino-4H-5,6-dihydro-1,2-oxazin als Öl.
- ¹H-NMR (CDCl₃):: δ = 4,9 (s,1H); 4,3 (2q,2H); 3,0 (m,2H); 2,7 (m,2H); 2,6 + 2,2 (AB-System,2H); 1,9 - 1,4 (m,14H); 1,4 (t,3H) ppm.

### b) 4,4-Pentamethylen-prolin-ethylester

1,6 g des Produkts aus Beispiel 4a) werden analog der in Beispiel 1b) angegebenen Vorschrift mit 0,2 g Raney-Nickel hydriert. Man erhält 0.9 g blaßgelbes Öl.
- ¹H-NMR (CDCl₃):: δ = 4,2 (q,2H); 3,9 (m,1H); 3,4 (m,2H); 1,9 - 1,3 (m,12H); 1,25 (t,3H) ppm.

## Patentansprüche (Patentansprüche für folgende(n) Vertragsstaat(en): AT, BE, CH, DE, FR, GB, GR, IT, LI, LU, NL, SE)

1. Verfahren zur Herstellung von Verbindungen der Formel I 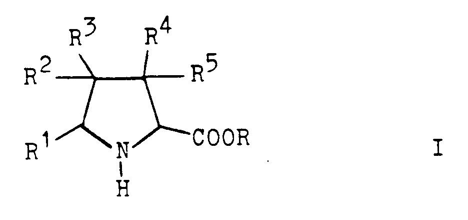 in welcher
R für Wasserstoff, (C₁-C₆)-Alkyl oder (C₇-C₉)-Aralkyl steht, und
R¹ bis R⁵ gleich oder verschieden sind und unabhängig voneinander Wasserstoff, (C₁-C₈)-Alkyl, (C₃-C₉)-Cycloalkyl, (C₃-C₉)-Cycloalkyl-(C₁-C₄)-alkyl, (C₅-C₉)-Cycloalkenyl-(C₁-C₄)-alkyl, (C₆-C₁₂)-Aryl-(C₁-C₄)-alkyl oder (C₆-C₁₂)-Aryl, wobei die beiden letztgenannten Substituenten jeweils im Arylteil durch (C₁-C₄)-Alkyl, (C₁-C₄)-Alkoxy, Hydroxy, Halogen, Nitro, Methylendioxy und/oder Cyano mono-, di- oder trisubstituiert sein können, bedeuten
oder in welcher
jeweils die Reste R¹ und R², R² und R³ sowie R⁴ und R⁵ zusammen mit dem sie tragenden Kohlenstoffatom bzw. mit den beiden sie tragenden Kohlenstoffatomen ein 4- bis 10-gliedriges gesättigtes oder ungesättigtes mono- oder bicyclisches carbocyclisches Ringsystem bilden und die übrigen Reste Wasserstoff sind,
das dadurch gekennzeichnet ist, daß man eine Verbindung der Formel II 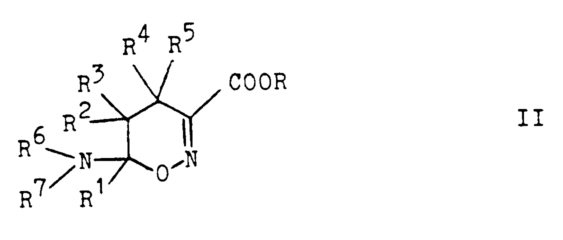 in welcher
R und R¹ bis R⁵ gleich oder verschieden sind und die obengenannte Bedeutung haben, und
R⁶ und R⁷ (C₁-C₆)-Alkyl, (C₇-C₉)-Aralkyl bedeuten, oder
R⁶ und R⁷ zusammen mit dem sie tragenden Stickstoffatom einen 5- bis 10-gliedrigen Heterocyclus bilden, der zusätzlich ein Sauerstoffatom enthalten kann,
bei Temperaturen zwischen -20°C und dem Siedepunkt des Reaktionsgemisches und einem H₂-Druck bei der Reaktion von 1 · 10⁵ bis 2 · 10⁷ N·m⁻² mit einem Reduktionsmittel behandelt, wobei als Reduktionsmittel Wasserstoff in Gegenwart eines Katalysators wie Palladiummohr, Palladium auf Aktivkohle, Platin auf Aktivkohle, Rhodium auf Aktivkohle, Raney-Nickel oder Raney-Kobalt sowie Natriumborhydrid, Natriumcyanborhydrid, sulfitiertes Natriumborhydrid (NaBH₂S₃), Boran-Dimethylsulfid, Boran-Pyridin, Boran-Trimethylamin, Natriumdithionit, Natrium in einem niederen Alkohol, Natriumamalgam oder Aluminiumamalgam, eingesetztwird, und gegebenenfalls unter hydrogenolytischen, sauren oder basischen Bedingungen in Verbindungen der Formel I, worin R Wasserstoff bedeutet, überführt.

2. Verfahren gemäß Anspruch 1, dadurch gekennzeichnet, daß
R wie in Anspruch 1 definiert ist,
R¹ bis R⁵ gleich oder verschieden sind und unabhängig voneinander Wasserstoff, Methyl, Ethyl, Propyl, Isopropyl, tert. Butyl, n-Pentyl, Cyclopentyl, Cyclohexyl, Cycloheptyl, Cyclopentylmethyl, Cyclopentylethyl, Cyclohexylmethyl, Phenyl, Naphthyl, 4-Methoxyphenyl, 4-Fluorphenyl, 4-Chlorphenyl, 3,4-Dimethoxyphenyl, 3,4-Methylendioxyphenyl, 3,4-Dichlorphenyl, p-Tolyl, Benzyl, 4-Methoxybenzyl, 4-Chlorbenzyl, Phenethyl, 2-Phenylpropyl oder 1-Phenylpropyl bedeuten,
oder in welcher
jeweils die Reste R¹ und R², R² und R³ sowie R⁴ und R⁵ in der oben definierten Weise einen Cyclobutan-, Cyclopentan-, Cyclohexan-, Cycloheptan-, Cyclooctan-, Bicyclo[2.2.1]heptan- oder Bicyclo[2.2.2]octan-Ring bilden, wobei die übrigen Reste Wasserstoff sind.

3. Verfahren gemäß einem oder mehreren der Ansprüche 1 und 2, dadurch gekennzeichnet, daß
R wie in Anspruch 1 definiert ist, insbesondere aber Wasserstoff, tert. Butyl oder Benzyl bedeutet und worin
einer oder zwei der Reste R¹ bis R⁵ unabhängig voneinander Methyl, Ethyl, Propyl, Isopropyl, n-Pentyl, Cyclopentyl, Cyclohexyl, Phenyl, 4-Methoxyphenyl, 4-Fluorphenyl, Benzyl, Phenethyl oder 4-Methoxybenzyl und die übrigen Wasserstoff bedeuten,
oder
jeweils die Reste R¹ und R² sowie R² und R³ mit dem sie tragenden Kohlenstoffatom bzw. mit den beiden sie tragenden Kohlenstoffatomen einen Cyclopentan-, Cyclohexan-, Cycloheptan, Bicyclo[2.2.1]heptan- oder Bicyclo[2.2.2]octan-Ring bilden, wobei die übrigen Reste Wasserstoff bedeuten.

4. Verfahren gemäß einem oder mehreren der Ansprüche 1 bis 3 zur Herstellung von Spiro[bicyclo[2.2.2]octan-2,3'-pyrrolidin]-5'-carbonsäure-ethylester.

5. Verbindungen der Formel II 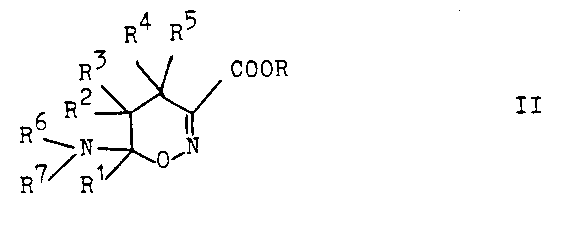 in welcher
R und R¹ bis R⁷ wie in Anspruch 1 definiert sind, mit der Maßgabe, daß nicht gleichzeitig R für Ethyl, R¹ und R² gemeinsam für eine -[CH₂]₄-Kette, R³ bis R⁵ für Wasserstoff und R⁶ und R⁷ mit dem sie tragenden Stickstoffatom für einen Morpholinring stehen.

6. Verfahren zur Herstellung von Verbindungen der Formel II gemäß Anspruch 5, dadurch gekennzeichnet, daß man eine Verbindung der Formel III 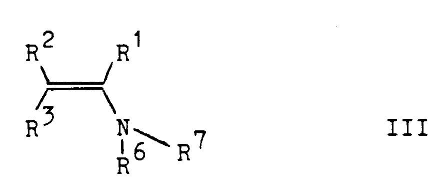 mit einer Verbindung der Formel IV 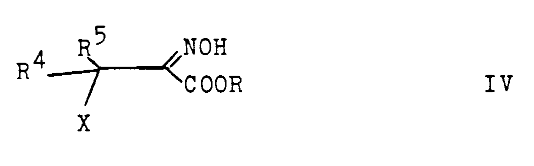 worin
R für Wasserstoff, (C₁-C₆)-Alkyl oder (C₇-C₉)-Aralkyl steht
R¹ bis R⁵ gleich oder verschieden sind und unabhängig voneinander Wasserstoff, (C₁-C₈)-Alkyl, (C₃-C₉)-Cycloalkyl, (C₃-C₉)-Cycloalkyl-(C₁-C₄)-alkyl, (C₅-C₉)-Cycloalkenyl-(C₁-C₄)-alkyl, (C₆-C₁₂)-Aryl-(C₁-C₄)-alkyl oder (C₆-C₁₂)-Aryl, wobei die beiden letztgenannten Substituenten jeweils im Arylteil durch (C₁-C₄)-Alkyl, (C₁-C₄)-Alkoxy, Hydroxy, Halogen, Nitro, Methylendioxy und/oder Cyano mono-, di- oder trisubstituiert sein können, bedeuten
oder worin
jeweils die Reste R¹ und R², R² und R³ sowie R⁴ und R⁵ zusammen mit dem sie tragenden Kohlenstoffatom bzw. mit den beiden sie tragenden Kohlenstoffatomen ein 4- bis 10-gliedriges gesättigtes oder ungesättigtes mono- oder bicyclisches carbocyclisches Ringsystem bilden und die übrigen Reste Wasserstoff sind,
R⁶ und R⁷ (C₁-C₄)-Alkyl, (C₇-C₉)-Aralkyl bedeuten oder
R⁶ und R⁷ zusammen mit dem sie tragenden Stickstoffatom einen 5- bis 10-gliedrigen Heterocyclus bilden, der zusätzlich ein Sauerstoffatom enthalten kann, und
X Chlor oder Brom bedeutet, umsetzt.

## Patentansprüche (Patentansprüche für folgende(n) Vertragsstaat(en): ES)

1. Verfahren zur Herstellung von Verbindungen der Formel I 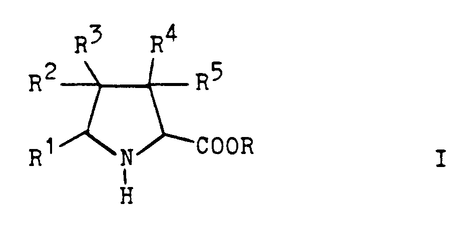 in welcher
R für Wasserstoff, (C₁-C₆)-Alkyl oder (C₇-C₉)-Aralkyl steht, und
R¹ bis R⁵ gleich oder verschieden sind und unabhängig voneinander Wasserstoff, (C₁-C₈)-Alkyl, (C₃-C₉)-Cycloalkyl, (C₃-C₉)-Cycloalkyl-(C₁-C₄)-alkyl, (C₅-C₉)-Cycloalkenyl-(C₁-C₄)-alkyl, (C₆-C₁₂)-Aryl-(C₁-C₄)-alkyl oder (C₆-C₁₂)-Aryl, wobei die beiden letztgenannten Substituenten jeweils im Arylteil durch (C₁-C₄)-Alkyl, (C₁-C₄)-Alkoxy, Hydroxy, Halogen, Nitro, Methylendioxy und/oder Cyano mono-, di- oder trisubstituiert sein können, bedeuten
oder in welcher
jeweils die Reste R¹ und R², R² und R³ sowie R⁴ und R⁵ zusammen mit dem sie tragenden Kohlenstoffatom bzw. mit den beiden sie tragenden Kohlenstoffatomen ein 4- bis 10-gliedriges gesättigtes oder ungesättigtes mono- oder bicyclisches carbocyclisches Ringsystem bilden und die übrigen Reste Wasserstoff sind,
das dadurch gekennzeichnet ist, daß man eine Verbindung der Formel II 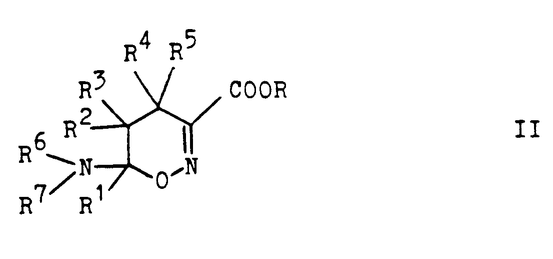 in welcher
R und R¹ bis R⁵ gleich oder verschieden sind und die obengenannte Bedeutung haben, und
R⁶ und R⁷ (C₁-C₆)-Alkyl, (C₇-C₉)-Aralkyl bedeuten, oder
R⁶ und R⁷ zusammen mit dem sie tragenden Stickstoffatom einen 5- bis 10-gliedrigen Heterocyclus bilden, der zusätzlich ein Sauerstoffatom enthalten kann,
bei Temperaturen zwischen -20°C und dem Siedepunkt des Reaktionsgemisches und einem H₂-Druck bei der Reaktion von 1 · 10⁵ bis 2 · 10⁷ N·m⁻² mit einem Reduktionsmittel behandelt, wobei als Reduktionsmittel Wasserstoff in Gegenwart eines Katalysators wie Palladiummohr, Palladium auf Aktivkohle, Platin auf Aktivkohle, Rhodium auf Aktivkohle, Raney-Nickel oder Raney-Kobalt sowie Natriumborhydrid, Natriumcyanborhydrid, sulfitiertes Natriumborhydrid (NaBH₂S₃), Boran-Dimethylsulfid, Boran-Pyridin, Boran-Trimethylamin, Natriumdithionit, Natrium in einem niederen Alkohol, Natriumamalgam oder Aluminiumamalgam, eingesetztwird, und gegebenenfalls unter hydrogenolytischen, sauren oder basischen Bedingungen in Verbindungen der Formel I, worin R Wasserstoff bedeutet, überführt.

2. Verfahren gemäß Anspruch 1, dadurch gekennzeichnet, daß
R wie in Anspruch 1 definiert ist,
R¹ bis R⁵ gleich oder verschieden sind und unabhängig voneinander Wasserstoff, Methyl, Ethyl, Propyl, Isopropyl, tert. Butyl, n-Pentyl, Cyclopentyl, Cyclohexyl, Cycloheptyl, Cyclopentylmethyl, Cyclopentylethyl, Cyclohexylmethyl, Phenyl, Naphthyl, 4-Methoxyphenyl, 4-Fluorphenyl, 4-Chlorphenyl, 3,4-Dimethoxyphenyl, 3,4-Methylendioxyphenyl, 3,4-Dichlorphenyl, p-Tolyl, Benzyl, 4-Methoxybenzyl, 4-Chlorbenzyl, Phenethyl, 2-Phenylpropyl oder 1-Phenylpropyl bedeuten,
oder in welcher
jeweils die Reste R¹ und R², R² und R³ sowie R⁴ und R⁵ in der oben definierten Weise einen Cyclobutan-, Cyclopentan-, Cyclohexan-, Cycloheptan-, Cyclooctan-, Bicyclo[2.2.1]heptan- oder Bicyclo[2.2.2]octan-Ring bilden, wobei die übrigen Reste Wasserstoff sind.

3. Verfahren gemäß einem oder mehreren der Ansprüche 1 und 2, dadurch gekennzeichnet, daß
R wie in Anspruch 1 definiert ist, insbesondere aber Wasserstoff, tert. Butyl oder Benzyl bedeutet und worin
einer oder zwei der Reste R¹ bis R⁵ unabhängig voneinander Methyl, Ethyl, Propyl, Isopropyl, n-Pentyl, Cyclopentyl, Cyclohexyl, Phenyl, 4-Methoxyphenyl, 4-Fluorphenyl, Benzyl, Phenethyl oder 4-Methoxybenzyl und die übrigen Wasserstoff bedeuten,
oder
jeweils die Reste R¹ und R² sowie R² und R³ mit dem sie tragenden Kohlenstoffatom bzw. mit den beiden sie tragenden Kohlenstoffatomen einen Cyclopentan-, Cyclohexan-, Cycloheptan-, Bicyclo[2.2.1]heptan- oder Bicyclo[2.2.2]octan-Ring bilden, wobei die übrigen Reste Wasserstoff bedeuten.

4. Verfahren gemäß einem oder mehreren der Ansprüche 1 bis 3 zur Herstellung von Spiro[bicyclo[2.2.2]octan-2,3'-pyrrolidin]-5'-carbonsäure-ethylester.

5. Verfahren zur Herstellung von Verbindungen der Formel II, 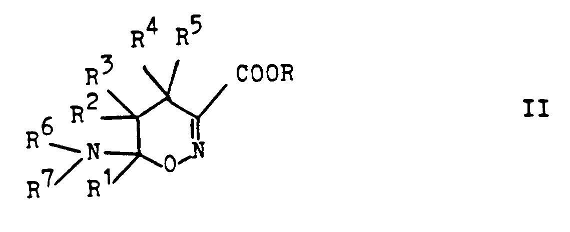 in welcher
R und R¹ bis R⁷ wie in Anspruch 1 definiert sind, mit der Maßgabe, daß nicht gleichzeitig R für Ethyl, R¹ und R² gemeinsam für eine -[CH₂]₄-Kette, R³ bis R⁵ für Wasserstoff und R⁶ und R⁷ mit dem sie tragenden Stickstoffatom für einen Morpholinring stehen,
dadurch gekennzeichnet, daß man eine Verbindung der Formel III 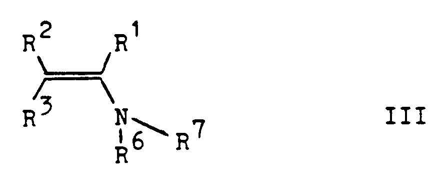 mit einer Verbindung der Formel IV 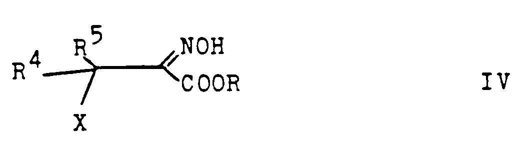 worin
R für Wasserstoff, (C₁-C₆)-Alkyl oder (C₇-C₉)-Aralkyl steht
R¹ bis R⁵ gleich oder verschieden sind und unabhängig voneinander Wasserstoff, (C₁-C₈)-Alkyl, (C₃-C₉)-Cycloalkyl, (C₃-C₉)-Cycloalkyl-(C₁-C₄)-alkyl, (C₅-C₉)-Cycloalkenyl-(C₁-C₄)-alkyl, (C₆-C₁₂)-Aryl-(C₁-C₄)-alkyl oder (C₆-C₁₂)-Aryl, wobei die beiden letztgenannten Substituenten jeweils im Arylteil durch (C₁-C₄)-Alkyl, (C₁-C₄)-Alkoxy, Hydroxy, Halogen, Nitro, Methylendioxy und/oder Cyano mono-, di- oder trisubstituiert sein können, bedeuten
oder worin
jeweils die Reste R¹ und R², R² und R³ sowie R⁴ und R⁵ zusammen mit dem sie tragenden Kohlenstoffatom bzw. mit den beiden sie tragenden Kohlenstoffatomen ein 4- bis 10-gliedriges gesättigtes oder ungesättigtes mono- oder bicyclisches carbocyclisches Ringsystem bilden und die übrigen Reste Wasserstoff sind,
R⁶ und R⁷ (C₁-C₄)-Alkyl, (C₇-C₉)-Aralkyl bedeuten oder
R⁶ und R⁷ zusammen mit dem sie tragenden Stickstoffatom einen 5- bis 10-gliedrigen Heterocyclus bilden, der zusätzlich ein Sauerstoffatom enthalten kann, und
X Chlor oder Brom bedeutet, umsetzt.

## Claims (Claims for the following Contracting State(s): AT, BE, CH, DE, FR, GB, GR, IT, LI, LU, NL, SE)

1. A process for preparing compounds of the formula I 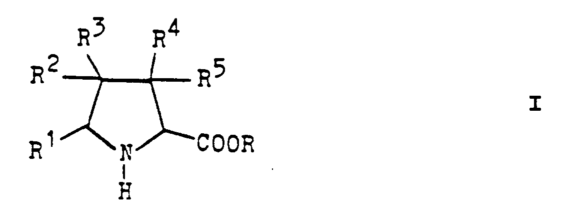 in which
R stands for hydrogen, (C₁-C₆)-alkyl or (C₇-C₉)-aralkyl, and
R¹ to R⁵ are identical or different and independently of one another denote hydrogen, (C₁-C₈)-alkyl, (C₃-C₉)-cycloalkyl, (C₃-C₉)-cycloalkyl-(C₁-C₄)-alkyl, (C₅-C₉)-cycloalkenyl-(C₁-C₄)-alkyl, (C₆-C₁₂)-aryl-(C₁-C₄)-alkyl or (C₆-C₁₂)-aryl, where the two last-mentioned substituents can each be mono-, di- or trisubstituted in the aryl moiety by (C₁-C₄)-alkyl, (C₁-C₄)-alkoxy, hydroxyl, halogen, nitro, methylenedioxy and/or cyano,
or in which
the pairs of radicals R¹ and R², R² and R³, and R⁴ and R⁵ together with the carbon atom or two carbon atoms supporting them form a 4- to 10-membered saturated or unsaturated mono- or bicyclic carbocyclic ring system and the other radicals are hydrogen,
which comprises treating a compound of the formula II 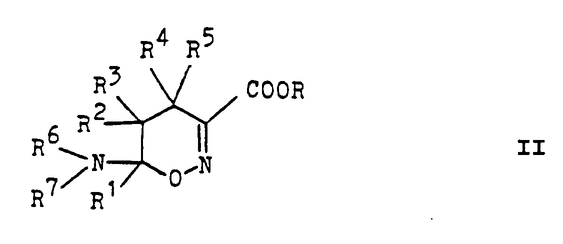 in which
R and R¹ to R⁵ are identical or different and have the abovementioned meaning, and
R⁶ and R⁷ denote (C₁-C₆)-alkyl or (C₇-C₉)-aralkyl or
R⁶ and R⁷ together with the nitrogen atom supporting them form a 5- to 10-membered heterocycle which can additionally contain an oxygen atom,
at temperatures between -20°C and the boiling point of the reaction mixture and at an H₂ pressure of from 1·10⁵ to 2·10⁷ N·m⁻² with a reducing agent, the reducing agent used being hydrogen in the presence of a catalyst such as palladium black, palladium on active carbon, platinum on active carbon, rhodium on active carbon, Raney nickel or Raney cobalt, sodium borohydride, sodium cyanoborohydride, sulfited sodium borohydride (NaBH₂S₃), borane/dimethyl sulfide, borane/pyridine, borane/trimethylamine, sodium dithionite, sodium in a lower alcohol, sodium amalgam or aluminum amalgam, and if desired converting the product under hydrogenolytic, acid or basic conditions into compounds of the formula I in which R is hydrogen.

2. The process as claimed in claim 1, wherein R is defined as in claim 1,
R¹ to R⁵ are identical or different and independently of one another denote hydrogen, methyl, ethyl, propyl, isopropyl, tert-butyl, n-pentyl, cyclopentyl, cyclohexyl, cycloheptyl, cyclopentylmethyl, cyclopentylethyl, cyclohexylmethyl, phenyl, naphthyl, 4-methoxyphenyl, 4-fluorophenyl, 4-chlorophenyl, 3,4-dimethoxyphenyl, 3,4-methylenedioxyphenyl, 3,4-dichlorophenyl, p-tolyl, benzyl, 4-methoxybenzyl, 4-chlorobenzyl, phenethyl, 2-phenylpropyl or 1-phenylpropyl
or in which
the pairs of radicals R¹ and R², R² and R³, and R⁴ and R⁵ form in the above-defined manner a cyclobutane, cyclopentane, cyclohexane, cycloheptane, cyclooctane, bicyclo[2.2.1]heptane or bicyclo[2.2.2]octane ring, the remaining radicals being hydrogen.

3. The process as claimed in one or more of claims 1 and 2, wherein
R is as defined in claim 1, but denotes in particular hydrogen, tert-butyl or benzyl, and in which one or two of the radicals R¹ to R⁵ independently of each other denote methyl, ethyl, propyl, isopropyl, n-pentyl, cyclopentyl, cyclohexyl, phenyl, 4-methoxyphenyl, 4-fluorophenyl, benzyl, phenethyl or 4-methoxybenzyl and the others denote hydrogen, or
the pairs of radicals R¹ and R², and R² and R³ together with the carbon atom or two carbon atoms supporting them form a cyclopentane, cyclohexane, cycloheptane, bicyclo[2.2.1]-heptane or bicyclo[2.2.2]octane ring, the other radicals denoting hydrogen.

4. The process as claimed in one or more of claims 1 to 3 for preparing ethyl spiro[bicyclo[2.2.2]octane-2,3'-pyrrolidine]-5,-carboxylate.

5. A compound of the formula II 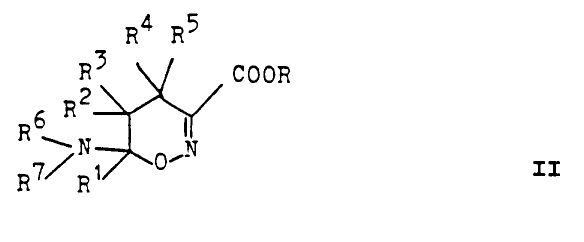 in which
R and R¹ to R⁷ are as defined in claim 1, with the proviso that simultaneously R does not stand for ethyl, R¹ and R² together do not stand for a -[CH₂]₄-chain, R³ to R⁵ do not stand for hydrogen and R⁶ and R⁷ together with the nitrogen atom supporting them do not stand for a morpholine ring.

6. A process for preparing compounds of the formula II as claimed in claim 5, which comprises reacting a compound of the formula III 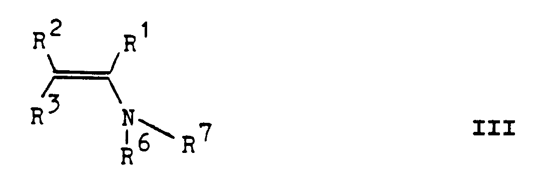 with a compound of the formula IV 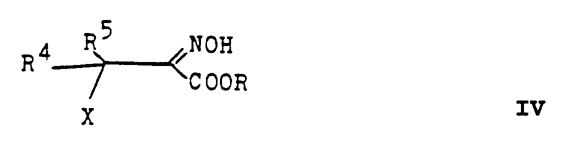 in which
R stands for hydrogen, (C₁-C₆)-alkyl or (C₇-C₉)-aralkyl,
R¹ to R⁵ are identical or different and independently of one another denote hydrogen, (C₁-C₈)-alkyl, (C₃-C₉)-cycloalkyl, (C₃-C₉)-cycloalkyl-(C₁-C₄)-alkyl, (C₅-C₉)-cycloalkenyl-(C₁-C₄)-alkyl, (C₆-C₁₂)-aryl-(C₁-C₄)-alkyl or (C₆-C₁₂)-aryl, where the two last-mentioned substituents can each be mono-, di- or trisubstituted in the aryl moiety by (C₁-C₄)-alkyl, (C₁-C₄)-alkoxy, hydroxyl, halogen, nitro, methylenedioxy and/or cyano,
or in which
the pairs of radicals R¹ and R², R² and R³, and R⁴ and R⁵ together with the carbon atom or two carbon atoms supporting them form a 4- to a 10-membered saturated or unsaturated mono- or bicyclic carbocyclic ring system and the other radicals are hydrogen,
R⁶ and R⁷ denote (C₁-C₄) alkyl or (C₇-C₉)-aralkyl or
R⁶ and R⁷ together with the nitrogen atom supporting them form a 5- to 10-membered heterocycle which can additionally contain an oxygen atom, and
X denotes chlorine or bromine.

## Claims (Claims for the following Contracting State(s): ES)

1. A process for preparing compounds of the formula I 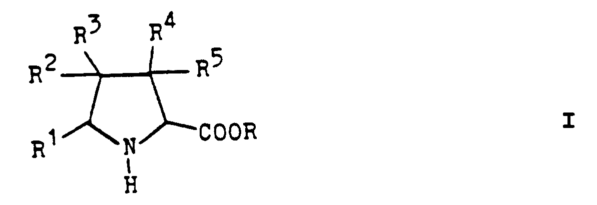 in which
R stands for hydrogen, (C₁-C₆)-alkyl or (C₇-C₉)-aralkyl, and
R¹ to R⁵ are identical or different and independently of one another denote hydrogen, (C₁-C₈)-alkyl, (C₃-C₉)-cycloalkyl, (C₃-C₉)-cycloalkyl-(C₁-C₄)-alkyl, (C₅-C₉)-cycloalkenyl-(C₁-C₄)-alkyl, (C₆-C₁₂)-aryl-(C₁-C₄)-alkyl or (C₆-C₁₂)-aryl, where the two last-mentioned substituents can each be mono-, di- or trisubstituted in the aryl moiety by (C₁-C₄)-alkyl, (C₁-C₄)-alkoxy, hydroxyl, halogen, nitro, methylenedioxy and/or cyano,
or in which
the pairs of radicals R¹ and R², R² and R³, and R⁴ and R⁵ together with the carbon atom or two carbon atoms supporting them form a 4- to 10-membered saturated or unsaturated mono- or bicyclic carbocyclic ring system and the other radicals are hydrogen,
which comprises treating a compound of the formula II 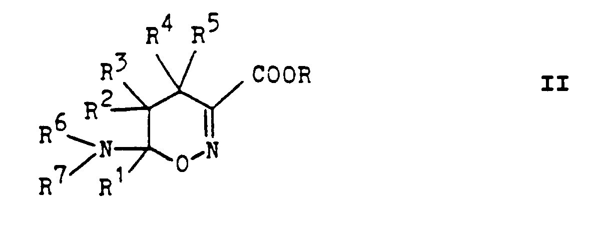 in which
R and R¹ to R⁵ are identical or different and have the abovementioned meaning, and
R⁶ and R⁷ denote (C₁-C₆)-alkyl or (C₇-C₉)-aralkyl or
R⁶ and R⁷ together with the nitrogen atom supporting them form a 5- to 10-membered heterocycle which can additionally contain an oxygen atom,
at temperatures between -20°C and the boiling point of the reaction mixture and at an H₂ pressure of from 1·10⁵ to 2·10⁷ N·m⁻² with a reducing agent, the reducing agent used being hydrogen in the presence of a catalyst such as palladium black, palladium on active carbon, platinum on active carbon, rhodium on active carbon, Raney nickel or Raney cobalt, sodium borohydride, sodium cyanoborohydride, sulfited sodium borohydride (NaBH₂S₃), borane/dimethyl sulfide, borane/pyridine, borane/trimethylamine, sodium dithionite, sodium in a lower alcohol, sodium amalgam or aluminum amalgam, and if desired converting the product under hydrogenolytic, acid or basic conditions into compounds of the formula I in which R is hydrogen.

2. The process as claimed in claim 1, wherein R is defined as in claim 1,
R¹ to R⁵ are identical or different and independently of one another denote hydrogen, methyl, ethyl, propyl, isopropyl, tert-butyl, n-pentyl, cyclopentyl, cyclohexyl, cycloheptyl, cyclopentylmethyl, cyclopentylethyl, cyclohexylmethyl, phenyl, naphthyl, 4-methoxyphenyl, 4-fluorophenyl, 4-chlorophenyl, 3,4-dimethoxyphenyl, 3,4-methylenedioxyphenyl, 3,4-dichlorophenyl, p-tolyl, benzyl, 4-methoxybenzyl, 4-chlorobenzyl, phenethyl, 2-phenylpropyl or 1-phenylpropyl
or in which
the pairs of radicals R¹ and R², R³ and R³, and R⁴ and R⁵ form in the above-defined manner a cyclobutane, cyclopentane, cyclohexane, cycloheptane, cyclooctane, bicyclo[2.2.1]heptane or bicyclo[2.2.2]octane ring, the remaining radicals being hydrogen.

3. The process as claimed in one or more of claims 1 and 2, wherein
R is as defined in claim 1, but denotes in particular hydrogen, tert-butyl or benzyl, and in which one or two of the radicals R¹ to R⁵ independently of each other denote methyl, ethyl, propyl, isopropyl, n-pentyl, cyclopentyl, cyclohexyl, phenyl, 4-methoxyphenyl, 4-fluorophenyl, benzyl, phenethyl or 4-methoxybenzyl and the others denote hydrogen, or
the pairs of radicals R¹ and R², and R² and R³ together with the carbon atom or two carbon atoms supporting them form a cyclopentane, cyclohexane, cycloheptane, bicyclo[2.2.1]-heptane or bicyclo[2.2.2]octane ring, the other radicals denoting hydrogen.

4. The process as claimed in one or more of claims 1 to 3 for preparing ethyl spiro[bicyclo[2.2.2]octane-2,3'-pyrrolidine]-5'-carboxylate.

5. A process for preparing compounds of the formula II 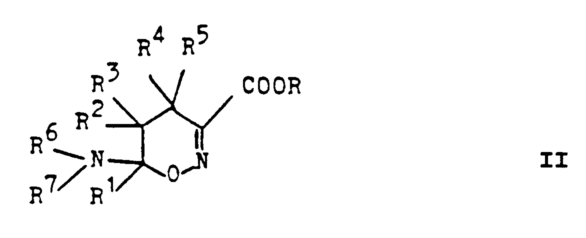 in which
R and R¹ to R⁷ are as defined in claim 1, with the proviso that simultaneously R does not stand for ethyl, R¹ and R² together do not stand for a -[CH₂]₄-chain, R³ to R⁵ do not stand for hydrogen and R⁶ and R⁷ together with the nitrogen atom supporting them do not stand for a morpholine ring, which comprises reacting a compound of the formula III 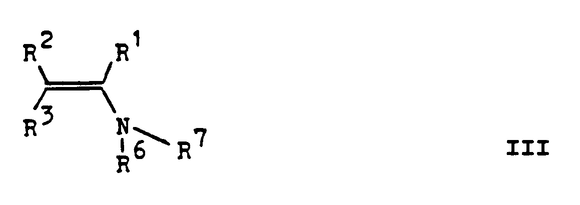 with a compound of the formula IV 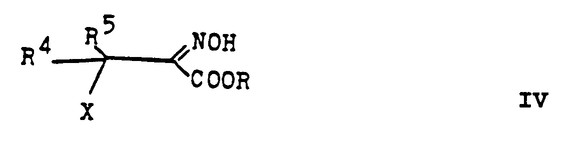 in which
R stands for hydrogen, (C₁-C₆)-alkyl or (C₇-C₉)-aralkyl,
R¹ to R⁵ are identical or different and independently of one another denote hydrogen, (C₁-C₈)-alkyl, (C₃-C₉)-cycloalkyl, (C₃-C₉)-cycloalkyl-(C₁-C₄)-alkyl, (C₅-C₉)-cycloalkenyl-(C₁-C₄)-alkyl, (C₆-C₁₂)-aryl-(C₁-C₄)-alkyl or (C₆-C₁₂)-aryl, where the two last-mentioned substituents can each be mono-, di- or trisubstituted in the aryl moiety by (C₁-C₄)-alkyl, (C₁-C₄)-alkoxy, hydroxyl, halogen, nitro, methylenedioxy and/or cyano,
or in which
the pairs of radicals R¹ and R², R² and R³, and R⁴ and R⁵ together with the carbon atom or two carbon atoms supporting them form a 4- to a 10-membered saturated or unsaturated mono- or bicyclic carbocyclic ring system and the other radicals are hydrogen,
R⁶ and R⁷ denote (C₁-C₄) alkyl or (C₇-C₉)-aralkyl or
R⁶ and R⁷ together with the nitrogen atom supporting them form a 5- to 10-membered heterocycle which can additionally contain an oxygen atom, and
X denotes chlorine or bromine.

## Revendications (Revendications pour l'(les) Etat(s) contractant(s) suivant(s): AT, BE, CH, DE, FR, GB, GR, IT, LI, LU, NL, SE)

1. Procédé pour la préparation de composés de formule I 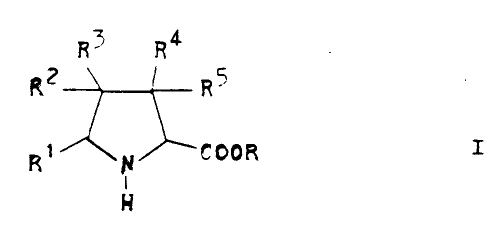 dans laquelle
R représente un atome d'hydrogène ou un radical alkyle en C₁-C₆ ou aralkyle en C₇-C₉, et
R¹ à R⁵ sont identiques ou différents et représentent, indépendamment les uns des autres, un atome d'hydrogène, un radical akyle en C₁-C₈, cycloalkyle en C₃-C₉, cycloalkyl(C₃-C₉)-alkyle(C₁-C₄), cycloalcényl(C₅-C₉)-alkyle-(C₁-C₄), aryl(C₆-C₁₂)-alkyle-(C₁-C₄) ou aryle en C₆-C₁₂, les deux substituants cités en dernier pouvant être mono-, di- ou trisubstitués chacun sur le fragement aryle par un ou des atomes d'halogène ou groupes alkyle en C₁-C₄, alcoxy en C₁-C₄, hydroxy, nitro, méthylènedioxy et/ou cyano,
ou dans laquelle
les radicaux R¹ et R², R² et R³ ainsi que R⁴ et R⁵ forment respectivement, avec l'atome de carbone qui les porte, ou avec les deux atomes de carbone qui les portent, un système cyclique carbocyclique mono- ou bicyclique à 4-10 chaînons, saturé ou insaturé, et les autres radicaux sont des atomes d'hydrogène,
caractérisé en ce que l'on traite par un réducteur un composé de formule II 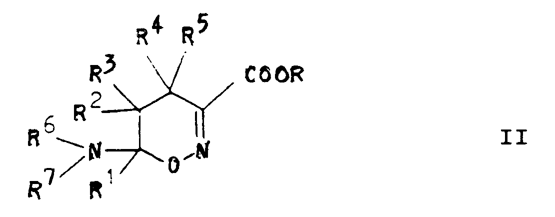 dans laquelle
R et R¹ à R⁵ sont identiques ou différents et ont les significations données plus haut, et
R⁶ et R⁷ représentent un radical alkyle en C₁-C₆ ou aralkyle en C₇-C₉, ou
R⁶ et R⁷ forment, conjointement avec l'atome d'azote qui les porte, un hétérocycle à 5-10 chaînons, qui peut contenir en outre un atome d'oxygène,
à des températures comprises entre -20°C et le point d'ébullition du mélange réactionnel et sous une pression d'hydrogène, dans la réaction, dans la plage de 1·10⁵ à 2·10⁷ N·m⁻², en utilisant comme réducteur de l'hydrogène en présence d'un catalyseur tel que le noir de palladium, le palladium sur charbon actif, le platine sur charbon actif, le rhodium sur charbon actif, le nickel de Raney ou le cobalt de Raney, ainsi que le borohydrure de sodium, le cyanoborohydrure de sodium, le borohydrure de sodium sulfité (NaBH₂S₃), le borane-diméthylsulfure, le borane-pyridine, le borane-triméthylamine, le dithionite de sodium, le sodium dans un alcool inférieur, l'amalgame de sodium ou l'amalgame d'aluminium, et éventuellement on les convertit, dans des conditions d'hydrogénolyse acides ou basiques, en composés de formule I dans lesquels R représente un atome d'hydrogène.

2. Procédé selon la revendication 1, caractérisé en ce que
R est tel que défini dans la revendication 1,
R¹ à R⁵ sont identiques ou différents et représentent, indépendamment les uns des autres, un atome d'hydrogène, le groupe méthyle, éthyle, propyle, isopropyle, tert-butyle, n-pentyle, cyclopentyle, cyclohexyle, cycloheptyle, cyclopentylméthyle, cyclopentyléthyle, cyclohexylméthyle, phényle, naphtyle, 4-méthoxyphényle, 4-fluorophényle, 4-chlorophényle, 3,4-diméthoxyphényle, 3,4-méthylènedioxyphényle, 3,4-dichlorophényle, p-tolyle, benzyle, 4-méthoxybenzyle, 4-chlorobenzyle, phénéthyle, 2-phénylpropyle ou 1-phénylpropyle, ou
les radicaux R¹ et R², R² et R³ ainsi que R⁴ et R⁵ forment respectivement, de la façon définie plus haut, un cycle cyclobutane, cyclopentane, cyclohexane, cycloheptane, cyclooctane, bicyclo[2.2.1]heptane ou bicyclo[2.2.2]octane, les autres radicaux étant des atomes d'hydrogène.

3. Procédé selon une ou plusieurs des revendications 1 et 2, caractérisé en ce que
R est tel que défini dans la revendication 1, mais représente en particulier un atome d'hydrogène ou le groupe tert-butyle ou benzyle,
et
un ou deux des radicaux R¹ à R⁵, indépendamment l'un de l'autre, représente(nt) le groupe méthyle, éthyle, propyle, isopropyle, n-pentyle, cyclopentyle, cyclohexyle, phényle, 4-méthoxyphényle, 4-fluorophényle, benzyle, phénéthyle ou 4-méthoxybenzyle, et les autres représentent des atomes d'hydrogène, ou
les radicaux R¹ et R² ainsi que R² et R³ forment respectivement, avec l'atome de carbone qui les porte, ou avec les deux atomes de carbone qui les portent, un cycle cyclopentane, cyclohexane, cycloheptane, bicyclo[2.2.1]heptane ou bicyclo[2.2.2]octane, les autres radicaux représentant des atomes d'hydrogène.

4. Procédé selon une ou plusieurs des revendications 1 à 3, pour la préparation du spiro[bicyclo[2.2.2]octane-2,3'-pyrrolidine]-5'-carboxylate d'éthyle.

5. Composés de formule II 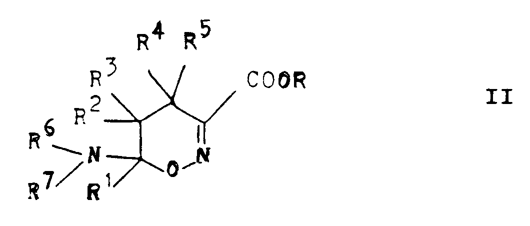 dans laquelle
R et R¹ à R⁷ sont tels que définis dans la revendication 1, étant entendu que les radicaux n'ont pas simultanément les significations suivantes: R celle du groupe éthyle, R¹ et R², ensemble, celle d'une chaîne -(CH₂)₄-, R³ à R⁵ celle d'atomes d'hydrogène et R⁶ et R⁷, avec l'atome d'azote qui les porte, celle d'un cycle morpholine.

6. Procédé pour la préparation de composés de formule II selon la revendication 5, caractérisé en ce que l'on fait réagir un composé de formule III 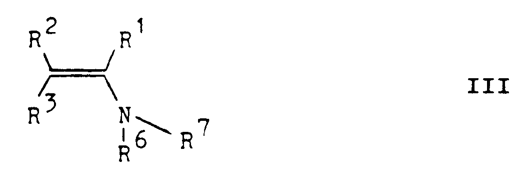 avec un composé de formule IV 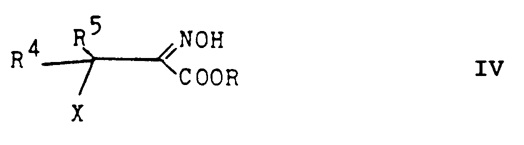 formules dans lesquelles
R représente un atome d'hydrogène ou un radical alkyle en C₁-C₆ ou aralkyle en C₇-C₉,
R¹ à R⁵ sont identiques ou différents et représentent, indépendamment les uns des autres, un atome d'hydrogène ou un radical alkyle en C₁-C₈, cycloalkyle en C₃-C₉, cycloalkyl(C₃-C₉)-alkyle(C₁-C₄), cycloalcényl(C₅-C₉)-alkyle-(C₁-C₄), aryl(C₆-C₁₂)-alkyle(C₁-C₄) ou aryle(C₆-C₁₂), les deux substituants cités en dernier pouvant être mono-, di- ou trisubstitués chacun sur le fragment aryle par un ou des atomes d'halogène ou groupes alkyle en C₁-C₄, alcoxy en C₁-C₄, hydroxy, nitro, méthylènedioxy et/ou cyano,
ou dans lesquels
les radicaux R¹ et R², R² et R³ ainsi que R⁴ et R⁵, respectivement, forment conjointement avec l'atome de carbone qui les porte, ou avec les deux atomes de carbone qui les portent, un cycle carbocyclique mono- ou bicyclique à 4-10 chaînons, saturé ou insaturé, et les autres radicaux sont des atomes d'hydrogène,
R⁶ et R⁷ représentent un radical alkyle en C₁-C₄ ou aralkyle en C₇-C₉, ou
R⁶ et R⁷ forment, conjointement avec l'atome d'azote qui les porte, un hétérocycle à 5-10 chaînons, qui peut contenir en outre un atome d'oxygène, et
X représente un atome de chlore ou de brome.

## Revendications (Revendications pour l'(les) Etat(s) contractant(s) suivant(s): ES)

1. Procédé pour la préparation de composés de formule I 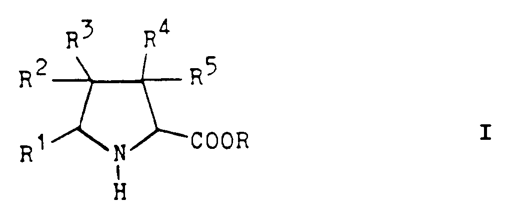 dans laquelle
R représente un atome d'hydrogène ou un radical alkyle en C₁-C₆ ou aralkyle en C₇-C₉, et
R¹ à R⁵ sont identiques ou différents et représentent, indépendamment les uns des autres, un atome d'hydrogène, un radical akyle en C₁-C₈, cycloalkyle en C₃-C₉, cycloalkyl(C₃-C₉)-alkyle(C₁-C₄), cycloalcényl(C₅-C₉)-alkyle-(C₁-C₄), aryl(C₆-C₁₂)-alkyle-(C₁-C₄) ou aryle en C₆-C₁₂, les deux substituants cités en dernier pouvant être mono-, di- ou trisubstitués chacun sur le fragement aryle par un ou des atomes d'halogène ou groupes alkyle en C₁-C₄, alcoxy en C₁-C₄, hydroxy, nitro, méthylènedioxy et/ou cyano,
ou dans laquelle
les radicaux R¹ et R², R² et R³ ainsi que R⁴ et R⁵ forment respectivement, avec l'atome de carbone qui les porte, ou avec les deux atomes de carbone qui les portent, un système cyclique carbocyclique mono- ou bicyclique à 4-10 chaînons, saturé ou insaturé, et les autres radicaux sont des atomes d'hydrogène,
caractérisé en ce que l'on traite par un réducteur un composé de formule II 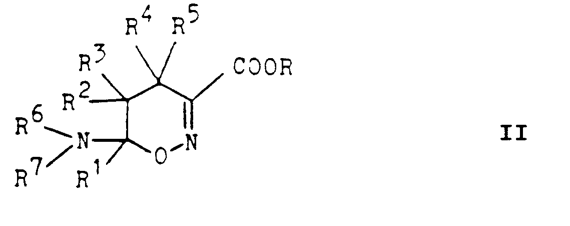 dans laquelle
R et R¹ à R⁵ sont identiques ou différents et ont les significations données plus haut, et
R⁶ et R⁷ représentent un radical alkyle en C₁-C₆ ou aralkyle en C₇-C₉, ou
R⁶ et R⁷ forment, conjointement avec l'atome d'azote qui les porte, un hétérocycle à 5-10 chaînons, qui peut contenir en outre un atome d'oxygène,
à des températures comprises entre -20°C et le point d'ébullition du mélange réactionnel et sous une pression d'hydrogène, dans la réaction, dans la plage de 1·10⁵ à 2·10⁷ N·m⁻², en utilisant comme réducteur de l'hydrogène en présence d'un catalyseur tel que le noir de palladium, le palladium sur charbon actif, le platine sur charbon actif, le rhodium sur charbon actif, le nickel de Raney ou le cobalt de Raney, ainsi que le borohydrure de sodium, le cyanoborohydrure de sodium, le borohydrure de sodium sulfité (NaBH₂S₃), le borane-diméthylsulfure, le borane-pyridine, le borane-triméthylamine, le dithionite de sodium, le sodium dans un alcool inférieur, l'amalgame de sodium ou l'amalgame d'aluminium, et éventuellement on les convertit, dans des conditions d'hydrogénolyse acides ou basiques, en composés de formule I dans lesquels R représente un atome d'hydrogène.

2. Procédé selon la revendication 1, caractérisé en ce que
R est tel que défini dans la revendication 1,
R¹ à R⁵ sont identiques ou différents et représentent, indépendamment les uns des autres, un atome d'hydrogène, le groupe méthyle, éthyle, propyle, isopropyle, tert-butyle, n-pentyle, cyclopentyle, cyclohexyle, cycloheptyle, cyclopentylméthyle, cyclopentyléthyle, cyclohexylméthyle, phényle, naphtyle, 4-méthoxyphényle, 4-fluorophényle, 4-chlorophényle, 3,4-diméthoxyphényle, 3,4-méthylènedioxyphényle, 3,4-dichlorophényle, p-tolyle, benzyle, 4-méthoxybenzyle, 4-chlorobenzyle, phénéthyle, 2-phénylpropyle ou 1-phénylpropyle, ou
les radicaux R¹ et R², R² et R³ ainsi que R⁴ et R⁵ forment respectivement, de la façon définie plus haut, un cycle cyclobutane, cyclopentane, cyclohexane, cycloheptane, cyclooctane, bicyclo[2.2.1]heptane ou bicyclo[2.2.2]octane, les autres radicaux étant des atomes d'hydrogène.

3. Procédé selon une ou plusieurs des revendications 1 et 2, caractérisé en ce que
R est tel que défini dans la revendication 1, mais représente en particulier un atome d'hydrogène ou le groupe tert-butyle ou benzyle,
et
un ou deux des radicaux R¹ à R⁵, indépendamment l'un de l'autre, représente(nt) le groupe méthyle, éthyle, propyle, isopropyle, n-pentyle, cyclopentyle, cyclohexyle, phényle, 4-méthoxyphényle, 4-fluorophényle, benzyle, phénéthyle ou 4-méthoxybenzyle, et les autres représentent des atomes d'hydrogène, ou
les radicaux R¹ et R² ainsi que R² et R³ forment respectivement, avec l'atome de carbone qui les porte, ou avec les deux atomes de carbone qui les portent, un cycle cyclopentane, cyclohexane, cycloheptane, bicyclo[2.2.1]heptane ou bicyclo[2.2.2]octane, les autres radicaux représentant des atomes d'hydrogène.

4. Procédé selon une ou plusieurs des revendications 1 à 3, pour la préparation du spiro[bicyclo[2.2.2]octane-2,3'-pyrrolidine]-5'-carboxylate d'éthyle.

5. Procédé pour la préparation de composés de formule II 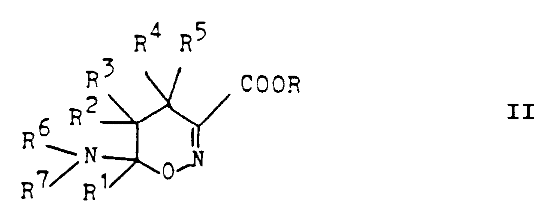 dans laquelle
R et R¹ à R⁷ sont tels que définis dans la revendication 1, étant entendu que les radicaux n'ont pas simultanément les significations suivantes: R celle du groupe éthyle, R¹ et R², ensemble, celle d'une chaîne -(CH₂)₄-, R³ à R⁵ celle d'atomes d'hydrogène et R⁶ et R⁷, avec l'atome d'azote qui les porte, celle d'un cycle morpholine,
et caractérisé en ce que l'on fait réagir un composé de formule III 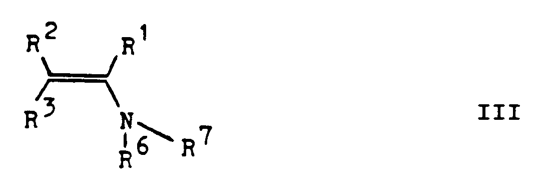 avec un composé de formule IV 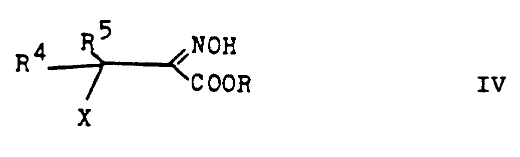 formules dans lesquelles
R représente un atome d'hydrogène ou un radical alkyle en C₁-C₆ ou aralkyle en C₇-C₉,
R¹ à R⁵ sont identiques ou différents et représentent, indépendamment les uns des autres, un atome d'hydrogène ou un radical alkyle en C₁-C₈, cycloalkyle en C₃-C₉, cycloalkyl(C₃-C₉)-alkyle(C₁-C₄), cycloalcényl(C₅-C₉)-alkyle-(C₁-C₄), aryl(C₆-C₁₂)-alkyle(C₁-C₄) ou aryle en C₆-C₁₂, les deux substituants cités en dernier pouvant être mono, di- ou trisubstitués chacun sur le fragment aryle par un ou des atomes d'halogène ou groupes alkyle en C₁-C₄, alcoxy en C₁-C₄, hydroxy, nitro, méthylènedioxy et/ou cyano,
ou dans lesquelles
les radicaux R¹ et R², R² et R³ ainsi que R⁴ et R⁵, respectivement, forment conjointement avec l'atome de carbone qui les porte, ou avec les deux atomes de carbone qui les portent, un cycle carbocyclique mono- ou bicyclique à 4-10 chaînons, saturé ou insaturé, et les autres radicaux sont des atomes d'hydrogène,
R⁶ et R⁷ représentent un radical alkyle en C₁-C₄ ou aralkyle en C₇-C₉, ou
R⁶ et R⁷ forment, conjointement avec l'atome d'azote qui les porte, un hétérocycle à 5-10 chaînons, qui peut contenir en outre un atome d'oxygène, et
X représente un atome de chlore ou de brome.
